# EUROPEAN PATENT SPECIFICATION

(11) **EP 0 506 920 B1**
(45) Date of publication and mention of the grant of the patent: **09.04.1997**
(21) Application number: 91918535.5
(22) Date of filing: 03.09.1991
(51) Int. Cl.: A61M 25/00, A61F 5/453, A61F 5/455, A61M 25/10

(54) **URINARY CONTROL WITH INFLATABLE SEAL**
URINKONTROLLE MIT AUFBLASBARER DICHTUNG
MAITRISE DE L'INCONTINENCE URINAIRE A L'AIDE D'UN JOINT GONFLABLE

(30) Priority: 22.10.1990 US 600629
(43) Date of publication of application: 07.10.1992
(73) Proprietor: SALAMA, Fouad A., West Des Moines, IA 50265 (US)
(72) Inventor: SALAMA, Fouad A., West Des Moines, IA 50265 (US)
(74) Representative: Desaix, Anne
(86) International application number: US9106228
(87) International publication number: WO9206731

(56) References cited:
- WO-A-82/03557
- US-A- 3 459 175
- US-A- 3 707 146
- US-A- 3 811 448
- US-A- 3 841 304
- US-A- 4 626 250
- US-A- 4 710 169
- US-A- 4 813 935

## Description

Incontinence is a problem for many people including older adults. Present day approaches to dealing with incontinence such as the Foley catheter often times causes urinary tract infection. A bag for urine is required and smell becomes a problem. The chances of infection are increased each time the bag is changed. The cost for the Foley catheters and pads is substantial. An inflatable conventional spherical balloon is used to keep the catheter in the bladder, but leakage around the catheter occurs and is a problem. It was not an object of this product to provide a seal around the catheter at the bladder orifice.

US-A-4.813.935 discloses a urinary control system comprising a balloon having a toroid shape when inflated. This shape provided a contact between the balloon and the bladder of the Foley catheter type.

WO-82/03557 discloses a urinary catheter balloon having a shape substantially corresponding to the shape of the bladder to prevent contact between a catheter tip and the inner wall of the bladder.

A urinary control system comprising an inner balloon is also known from US-A-384.304. However, in this system the balloon is spherical and has to be pulled tightly against the inner urethra opening.

The present invention aims to overcome the drawbacks of the prior art by providing a simple inexpensive device for controlling urine flow in the urethra, which is compatible to the body and will not cause discomfort, infection and pass urine only through operation of the valve rather than around the outside of the catheter.

More precisely, the present invention relates to a urinary control system comprising an air line having an inflatable balloon at one end and an air inlet at the opposite end, the balloon being adapted to be inserted in a bladder neck through an urethra orifice of an urethra, a urine tube positioned in parallel relationship with said line and adapted to receive urine from the bladder, and seal means connected to said line adapted to maintain said balloon inflated,
said urinary control system being characterized in that the balloon, when inflated, has a generally pear shape corresponding to the shape of the bladder at the urethra orifice to sealingly engage the neck of the bladder to provide a seal around the urine tube in the bladder neck.

A valve may be provided in the urinary tube outside of the urethra.

An anchoring collar of hydrogel may frictionally engage the exterior of the urinary tube and is then positioned against the outer end of the urethra to hold the balloon in tight sealing engagement with the neck of the bladder.

The urinary control of this invention when used by a male may include the additional use of a support shell around the penis to stabilize the urinary tube which extends through the penis. The anchoring collar of hydrogel is positioned against the outer end of the support shell. The shell is one piece but includes a plurality of sections to allow for fitting the support shell to penises of different sizes. A collar of hydrogel is also placed between the inner end of the shell sections and the pubic bone base of the penis. An accordion type section is included to give the shell flexibility in accommodating penises of different lengths and to permit them to be disposed at varying angles to the body.

A hypodermic syringe or the like may function as an air pump when its needle is inserted into the air tube to inflate the balloon.

### DESCRIPTION OF THE DRAWINGS

Figure 1 is a fragmentary perspective view of the urinary control with inflatable seal.

Figure 2 is a longitudinal cross-sectional view of the urinary control in the urethra of a female.

Figure 3 is an enlarged cross-sectional view of the structure indicated by the line 3 - 3 in Figure 2.

Figure 4 is a cross-sectional view taken along line 4 - 4 in Figure 2 showing the valve in a closed condition.

Figure 5 is a view similar to Figure 4 but showing the valve in an open condition.

Figure 6 is a view similar to Figure 2 but showing the urinary control in the urethra of a male.

### DESCRIPTION OF THE PREFERRED EMBODIMENT

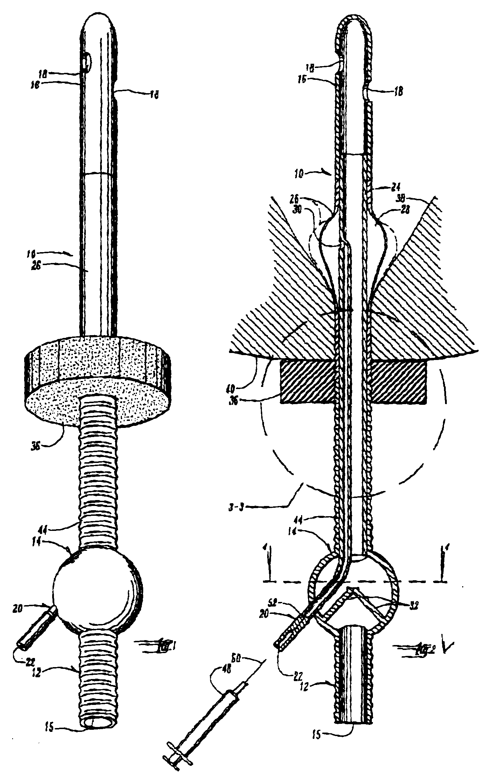

The urinary control of this invention is referred to generally by the reference numeral 10 as seen in Figure 1 and includes a urine tube 12 in which a valve 14 is connected. The tube 12 has an outer outlet end 15 and an inner inlet end 16 with sidewall openings 18.

An air tube 20 extends into the valve body 14 and along the length of the tube 12 towards the inlet end 16. The air tube 20 has an inlet end 22 and an outlet end 24 positioned in a balloon 26 formed in part by the sidewalls of the urine tube 14. Balloon chamber 28 is provided. The air tube outlet end includes an opening 30 in chamber 28.

The valve body 14 includes a pair of oppositely disposed blade elements 32 normally closed. Pressure on the opposite sidewalls of the valve body 14 will cause the valve elements 32 to spread as seen in Figure 5 and allow urine to flow toward the outlet end 15.

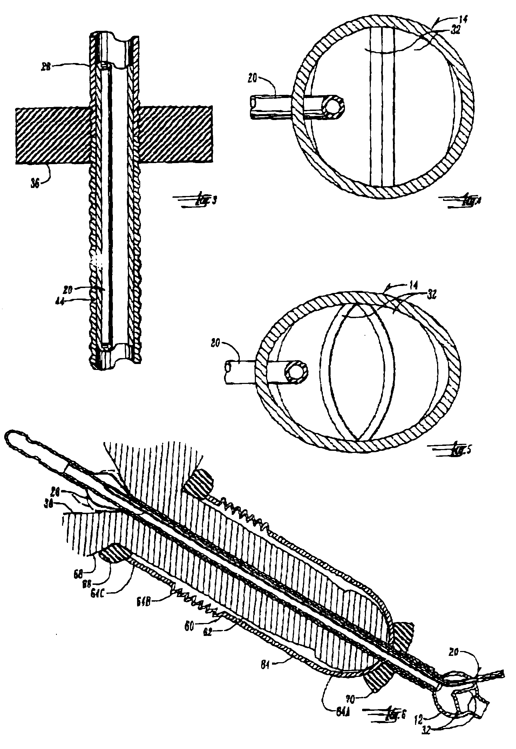

An anchoring collar means 36 of hydrogel is provided around the urine tube 12 outwardly of the balloon 26 and frictionally engages the outer surface of the urine tube 12 to hold the walls of the balloon 26 in tight sealing contact with the bladder orifice and neck 38 as seen in Figure 2. The balloon 26 is shaped to correspond to the shape of the inner side walls of the bladder at the orifice to provide a seal around the catheter thereby preventing leakage. This shape is generally pear shape. In the female, the collar 36 presses against the body around the opening to the urethra. A sleeve 42 also extends around the urine tube 12 and may be adjusted tightly against the collar 36 to assist in holding the collar tight against the person's body at the outlet end of the urethra. Rounded serrations 44 are provided along the outside of the urine tube 12 and register with serrations on the inside face of the collar 36 and serve to hold the collar 36 in place in turn holding the balloon seal 26 in place thereby preventing leakage around the tube 12.

A hypodermic syringe 48 functions as an air pump and has a needle 50 which is inserted into the inlet end 22 of the air tube 20. The inlet end 22 has a passageway 52 normally closed except when opened by the needle 50 thereby allowing air to be introduced into the tube to fill the balloon 26 but when the needle 50 is removed the passageway is sealed preventing air from escaping and deflating the balloon.

When the urinary control of this invention is used on a male, a one piece support shell 60 is provided around the penis 62 and includes an outer section 64 having an outer end 64A being rounded to the curvature of the head of the penis. An accordion pleats section 64B interconnects the outer section 64 with a base section 64C. The base section 64C presses against a hydrogel collar 66 which presses against the base (pubic bone) 68 of the penis. A second hydrogel collar 70 is positioned against the outer end of the rounded section 64A to hold the urine tube 12 in place such that the balloon 26 when inflated is pressed against the bladder neck 38. Among the properties of hydrogel is that it is soft and pliable but yet firm.

In use it is seen that the urine tube 12 will be inserted into the urethra of the male or female far enough that the balloon 26 will be seated in the neck 38 of the bladder. Air is introduced into the air tube 20 through the use of a hypodermic syringe. A hydrogel collar is then positioned against the body at the outer end of the urethra to hold the balloon 26 in position to form a seal with the bladder neck 38. The balloon is inflated from the solid line position in Figure 2 to the dash line inflated condition. When fully installed, no urine can leak around the urine tube 12 due to the seal the balloon 26 provides with the bladder neck 38. Urine can enter the openings 18 in the inlet end 16 of the urine tube 12 and pass into the valve 14 and upon actuation of the valve blades 32 by applying pressure to opposite sides of the valve 14, the valve will be open for drainage of the bladder through the outlet end 15.

The valve 14 and urine tube 12 are formed from elastomer silicone material of a 50 or 55 durometer from Dow Corning, Midland, Michigan. The balloon 26 may have a capacity of approximately 40 cc's.

## Claims

1. A urinary control system comprising an air line (20) having an inflatable balloon (26) at one end and an air inlet (22) at the opposite end, the balloon (26) being adapted to be inserted in a bladder neck (38) through an urethra orifice of an urethra, a urine tube (12) positioned in parallel relationship with said line (20) and adapted to receive urine from the bladder, and seal means (52) connected to said line adapted to maintain said balloon inflated,
said urinary control system being characterized in that the balloon (26), when inflated, has a generally pear shape corresponding to the shape of the bladder at the urethra orifice to sealingly engage the neck of the bladder (38) to provide a seal around the urine tube (12) in the bladder neck (38).

2. The system of claim 1 and an anchoring means (36) on said air line (20) adapted to operatively engage the body at the outer end of the urethra to hold said balloon (26) tight against the neck of the bladder (38).

3. The system of claim 1 or 2 wherein said urine tube is formed from elastomer silicone material.

4. The system of claim 2 wherein said anchoring means (36) includes a shell (60) adapted to fit over the penis (62), said shell having inner and outer ends (64A) with the inner end adapted to operatively engage the pubic bone base (68) of the penis (62).

5. The system of claim 4 wherein said shell (60) includes a flexible and expandable section (64B) adapted to fit penises of different sizes and disposed at varying angles to the body.

6. The system of claim 5 wherein said flexible and expandable section (64B) is further defined as including accordion pleats.

7. The system of claim 1 wherein said anchoring means is an anchoring collar (36,70) provided to extend around the urine tube for being positioned at the outer end of the urethra to hold the balloon (26) in tight sealing contact with the bladder office.

8. The system of claim 7 wherein said collar (36,70) urine tube (12) have cooperating serrations to provide a tight fit therebetween and thereby limit relative movement therebetween.

9. The system of claim 8 wherein said collar (36,70) is made of a soft pliable material.

10. The system of claim 9 wherein said collar (36,70) is made of hydrogel material.

## Patentansprüche

1. Urinsteuerungssystem, das eine Luftleitung (20) mit einem aufblasbaren Ballon (26) an einem Ende und einem Lufteinlaß (22) an dem gegenüberliegenden Ende aufweist, wobei der Ballon (26) zum Einsetzen in einen Harnblasenhals (38) durch eine Harnröhrenöffnung einer Harnröhre angepaßt ist, ferner weist das Urinsteuerungssystem eine parallel zu der Leitung (20) angeordnete und zum Aufnehmen von Urin aus der Blase angepaßte Urinröhre (12), und mit der Leitung verbundene Dichtmittel (52), die zum Erhalten des aufgeblasenen Zustands des Ballons ausgebildet sind, auf, wobei das Urinsteuerungssystem dadurch gekennzeichnet ist, daß der Ballon (26) im aufgeblasenen Zustand im wesentlichen eine Birnenform aufweist, die der Form der Blase an der Harnröhrenöffnung entspricht, um dichtend mit dem Harnblasenhals (38) zusammenzuwirken, um eine Dichtung um die Urinröhre (12) herum im Harnblasenhals (38) zu bilden.

2. Urinsteuerungssystem nach Anspruch 1, wobei Befestigungsmittel (36) an der Luftleitung (20) zum Zusammenwirken mit dem Körper an dem äußeren Ende der Harnröhre angepaßt sind, um den Ballon (26) fest gegen den Harnblasenhals (36) zu halten.

3. Urinsteuerungssystem nach Anspruch 1 oder 2, wobei der Urinröhre ist von Silicon Elastomere Material ausgemacht.

4. Urinsteuerungssystem nach Anspruch 2, wobei die Befestigungsmittel (36) eine über den Penis (62) passende Hülle (60) aufweisen, die innere und äußere Enden (64A) aufweist, wobei das innere Ende zum Zusammenwirken der Schambeinbasis (68) des Penis (62) ausgebildet ist.

5. Urinsteuerungssystem nach Anspruch 4, bei dem die Hülle (60) einen flexiblen und auseinanderziehbaren Bereich (64B) aufweist, der zum Anbringen auf unterschiedlich großen und in verschiedenen Winkeln zu dem Körper angeordneten Penissen ausgebildet ist.

6. Urinsteuerungssystem nach Anspruch 5, bei dem der flexible und auseinanderziehbare Bereich (64B) Ziehharmonikafalten umfaßt.

7. Urinsteuerungssystem nach Anspruch 1, bei dem das Befestigungsmittel eine Befestigungsmanschette (36, 70) ist, die sich um die Urinröhre herum erstreckt, um an dem äußeren Ende der Harnröhre angeordnet zu werden, um den Ballon (26) in fester dichtender Berührung mit dem Harnblasenhals zu halten.

8. Urinsteuerungssystem nach Anspruch 7, bei dem die Manschette (36, 70) und die Urinröhre (12) zusammenwirkende Riffelungen aufweisen, um einen festen Sitz aneinander zu bewirken und so relative Bewegungen zueinander zu begrenzen.

9. Urinsteuerungssystem nach Anspruch 8, bei dem die Manschette (36, 70) aus einem weichen nachgiebigen Material gefertigt ist.

10. Urinsteuerungssystem nach Anspruch 9, bei dem die Manschette (36, 70) aus einem Hydrogelmaterial gefertigt ist.

## Revendications

1. Système de maîtrise de l'incontinence urinaire comprenant une ligne d'air (20) ayant un ballon gonflable (26) à une extrémité et une entrée d'air (22) à l'extrémité opposée, le ballon (26) étant adapté pour être inséré dans un col de vessie (38) à travers un orifice urétéral d'un urètre, un tube pour l'urine (12) positionné parallèlement à ladite ligne (20) et adapté pour recevoir de l'urine provenant de la vessie, et un moyen de joint (52) connecté à ladite ligne adapté pour maintenir ledit ballon gonflé,
ledit système de maîtrise de l'incontinence urinaire étant caractérisé en ce que le ballon (26), une fois gonflé, a généralement une forme de poire, correspondant à la forme de la vessie au niveau de l'orifice urétéral pour engager par un joint étanche le col de la vessie (38) pour réaliser un joint étanche autour du tube pour l'urine (12) dans le col de la vessie (38).

2. Système selon la revendication 1, dans lequel un moyen d'ancrage (36) sur ladite ligne d'air (20) est adapté pour engager activement le corps au niveau de l'extrémité externe de l'urètre pour maintenir ledit ballon (26) fermement contre le col de la vessie (38).

3. Système selon la revendication 1 ou 2, dans lequel ledit tube pour l'urine est formé d'un matériau silicone élastomère.

4. Système selon la revendication 2, dans lequel ledit moyen d'ancrage (36) comporte une coquille (60) adaptée pour s'ajuster par-dessus le pénis (62), ladite coquille ayant des extrémités intérieure et extérieure (64A), l'extrémité intérieure étant adaptée pour engager activement la base osseuse pubienne (68) du pénis (62).

5. Système selon la revendication 4, dans lequel ladite coquille (60) comporte une section flexible et extensible (64B) adaptée pour s'ajuster à des pénis de différentes tailles et disposés suivant des angles variables par rapport au corps.

6. Système selon la revendication 5, dans lequel ladite section flexible et extensible (64B) est en outre définie comme comportant des plis en accordéon.

7. Système selon la revendication 1, dans lequel ledit moyen d'ancrage est une collerette d'ancrage (36, 70) prévue pour s'étendre autour du tube pour l'urine, à positionner au niveau de l'extrémité extérieure de l'urètre pour maintenir le ballon (26) fermement en contact à joint étanche avec l'orifice de la vessie.

8. Système selon la revendication 7, dans lequel ladite collerette (36, 70) et le tube pour l'urine (12) ont des indentations coopérantes pour fournir un ajustement serré entre eux et pour ainsi limiter tout mouvement relatif de l'un par rapport à l'autre.

9. Système selon la revendication 8, dans lequel ladite collerette (36, 70) est en matériau souple pliable.

10. Système selon la revendication 9, dans lequel ladite collerette (36, 70) est en matériau d'hydrogel.
